# EUROPEAN PATENT APPLICATION

(11) **EP 4 483 871 A1**
(43) Date of publication of application: **01.01.2025**
(21) Application number: 22927775.1
(22) Date of filing: 25.02.2022
(51) Int. Cl.: A61K 31/337, A61K 47/42, A61K 9/08, A61P 35/00

(54) **TAXOTERE COMPOSITION AND METHOD**

(71) Applicant: Zhuhai Beihai Biotech Co., Ltd., Zhuhai, Guangdong 519090 (CN)
(72) Inventor: SUN, Qun, Zhuhai, Guangdong 519090 (CN); JIANG, Haihua, Zhuhai, Guangdong 519090 (CN); WEI, Xiaohua, Zhuhai, Guangdong 519090 (CN); LIU, Jie, Zhuhai, Guangdong 519090 (CN)
(74) Representative: Kurig, Thomas
(86) International application number: PCT/CN2022/078020
(87) International publication number: WO 2023/159491

(57) **Abstract**

The present application provides pharmaceutical composition containing docetaxel and human serum albumin. Advantageously, the composition does not contain any polysorbate 80 surfactant, and may be used for infusing docetaxel to a patient at a dose of 75 mg/m². The composition is useful to treat cancer, for example, breast cancer, non-small cell lunch cancer, prostate cancer, gastric cancer, or head and neck cancer.

## Description

### TECHNICAL FIELD

The present invention relates to docetaxel formulations useful to treat cancer. In particular, the present invention relates to formulations containing docetaxel and human serum albumin ("HSA") that are useful to treat patients with breast cancer, non-small cell lung cancer, prostate cancer (such as castration-resistant prostate cancer), gastric cancer (such as adenocarcinoma), or head and neck cancer (such as squamous cell carcinoma of the head and neck).

### BACKGROUND

Many drugs for parenteral use are insoluble in water, and are thus formulated with solubilizing agents, surfactants, solvents, and/or emulsifiers that are irritating, allergenic, or toxic when administered to patients. *See, e.g.,* Briggs et al., Anesthesis 37, 1099 (1982), and Waugh et al., Am. J. Hasp. Pharmacists, 48, 1520 (1991)). Further, many of these drugs, especially those administered intravenously, cause undesirable side effects such as venous irritation, phlebitis, burning and pain on injection, venous thrombosis, extravasation, and other administration related side effects.

Taxanes play an important role in the treatment of various solid tumors. As a second-generation semi-synthetic taxane derivative, docetaxel is about twice as potent as paclitaxel in inhibiting microtubule depolymerization, and has the unique ability to alter certain classes of microtubules, which differs from most spindle poisons currently used in clinic. However, docetaxel has very poor water solubility. The clinical intravenous administration of commercially available two-vial formulation docetaxel (Taxotere^{®}) is formulated in a highly concentrated solution containing 40 mg docetaxel and 1040 mg Polysorbate 80 per mL. This concentrated solution must be carefully diluted with solvent containing 13% ethanol in saline before administration. It has been reported that docetaxel administration is associated with the occurrence of unpredictable (acute) hypersensitivity reactions and cumulative fluid retention. *See, e.g.,* Trudeau ME et al., J Clin Oncol 1996; 14:422-8, Piccart MJ et al., J Natl Cancer Inst 1995; 87:676-81, Bruno R et al., J Clin Oncol 1998; 16:187-96. These side-effects have been attributed, in part, to the presence of polysorbate 80. Polysorbate 80 is a synthetic nonionic surfactant used as an excipient in drug formulation. The reported rate of hypersensitivity reactions for docetaxel with polysorbate 80 (Taxotere) is estimated at 30% in patients who do not receive premedication. With premedication, the reported rate of hypersensitivity for docetaxel with polysorbate 80 (Taxotere) range from 8% to 13%. See Taxotere Prescribing Information. It has been reported that fatalities due to polysorbate 80-containing docetaxel anaphylaxis occurred after prophylaxis was given. This reinforces the importance of developing docetaxel formulations that do not contain polysorbate 80.

Currently no docetaxel product that does not contain polysorbate 80 has been approved for clinical use in the major pharmaceutical markets (e.g., USA, EU, China, and Japan). There is an urgent need to develop new docetaxel products containing no polysorbate 80 that have better safety profile for use in clinic. The formulations and methods described in the present invention help meet this need.

### SUMMARY

The current marketed formulation of Taxotere^{®} comprises either a one-vial formulation or a two-vial formulation (injection and diluent). The one-vial formulation contains a solution of docetaxel in polysorbate 80 and ethanol. The two-vial formulation contains a solution of docetaxel in polysorbate 80 and a solvent vial containing an aqueous solution of ethanol.

While both one-vial and two-vial forms of Taxotere have their advantages and disadvantages, both formulations contain a significant amount of polysorbate 80, which causes severe hypersensitivity reactions to some patients, and is associated with the most common adverse events of the Taxotere^{®} formulation. Advantageously, the formulations of this disclosure are prepared without any toxic surfactants, yet, they are bioequivalent to the commercial Taxotere^{®} formulation. As such, the formulations allow to achieve the therapeutic effect equal to or similar to that of the Taxotere^{®} formulation, while avoiding the adverse events that are associated with polysorbate surfactant in the Taxotere^{®} formulation. In particular, the formulation allow for dosing docetaxel at about 75 mg/m², for treating a variety of cancers, such as breast cancer, non-small cell lung cancer, prostate cancer, gastric cancer, or head and neck cancer. In addition, the formulations within the present claims contain only easily accessible, relatively inexpensive ingredients, such as human serum albumin, ethanol, and citric acid, all of which are separately approved by regulatory agencies, such as FDA or EMA, for pharmaceutical use.

WO2021/158632 describes formulations of docetaxel, human serum albumin, and ethanol.

In one general aspect, the present disclosure provides a pharmaceutical composition for infusion comprising docetaxel and human serum albumin,
wherein the composition is prepared by injecting a first liquid composition comprising docetaxel and ethanol into an infusion bag or bottle containing a second aqueous composition comprising human serum albumin in a parenterally acceptable vehicle,
wherein the composition does not contain polysorbate 80,
wherein the composition is administered by infusion to treat a patient diagnosed with a solid tumor selected from breast cancer, non-small cell lunch cancer, prostate cancer, gastric cancer, and head and neck cancer,
wherein the dose of docetaxel infused to said patient in need thereof is about 75 mg/m², and
wherein the composition is prepared within 24 hours prior to being infused to said patient.

In some embodiments, the composition is prepared within 4 hours prior to being infused to said patient.

In some embodiments, the first liquid composition is a sterile solution.

In some embodiments, the second aqueous composition is a sterile solution.

In some embodiments, the concentration of docetaxel in the composition is from about 0.05 mg/ml to about 0.5 mg/ml.

In some embodiments, the concentration of docetaxel in the composition is from about 0.1 mg/ml to about 0.4 mg/ml.

In some embodiments, the concentration of docetaxel in the composition is about 0.20 mg/ml.

In some embodiments, the concentration of docetaxel in the composition is about 0.25 mg/ml.

In some embodiments, the concentration of docetaxel in the composition is about 0.30 mg/ml.

In some embodiments, the concentration of human serum albumin in the composition is from about 0.01% to about 20% (w/v).

In some embodiments, the concentration of human serum albumin in the composition is from about 0.1% to about 10% (w/v).

In some embodiments, the concentration of human serum albumin in the composition is from about 0.2% to about 5% (w/v).

In some embodiments, the concentration of human serum albumin in the composition is from about 0.5% to about 2.5% (w/v).

In some embodiments, the composition is a clear aqueous infusion solution.

In some embodiments, the composition is administered repeatedly to said patient as a new cycle every 3 weeks.

In some embodiments, the composition is administered at a dose of 75 mg/m² of docetaxel.

In some embodiments, the composition is administered to treat said patient diagnosed with breast cancer.

In some embodiments, the composition is administered to treat said patient diagnosed with locally advanced or metastatic breast cancer.

In some embodiments, the composition is administered for adjuvant treatment of breast cancer.

In some embodiments, the composition is administered to treat said patient diagnosed with non-small cell lung cancer.

In some embodiments, the composition is administered to treat said patient diagnosed with locally advanced or metastatic non-small cell lung cancer.

In some embodiments, the composition is administered to treat said patient diagnosed with prostate cancer.

In some embodiments, the composition is administered to treat said patient diagnosed with metastatic castration-resistant prostate cancer.

In some embodiments, the composition is administered to treat said patient diagnosed with gastric cancer.

In some embodiments, the composition is administered to treat said patient diagnosed with advanced gastric adenocarcinoma, including the gastroesophageal junction.

In some embodiments, the composition is administered to treat said patient with head and neck cancer.

In some embodiments, the composition is administered for induction treatment of locally advanced squamous cell carcinoma of the head and neck.

Unless otherwise defined, all technical and scientific terms used herein have the same meaning as commonly understood by one of ordinary skill in the art to which the present application belongs. Methods and materials are described herein for use in the present application; other, suitable methods and materials known in the art can also be used. The materials, methods, and examples are illustrative only and not intended to be limiting. All publications, patent applications, patents, sequences, database entries, and other references mentioned herein are incorporated by reference in their entirety. In case of conflict, the present specification, including definitions, will control.

Other features and advantages of the present application will be apparent from the following detailed description and figures, and from the claims.

### DESCRIPTION OF DRAWINGS

FIG. 1 is a linear plot of mean concentration of total Docetaxel versus time curves after administration of BH009 and Winthrop (authorized generic Taxotere) (docetaxel) Injection to advanced solid tumor patients.
FIG. 2 is a linear plot of mean concentration of free Docetaxel versus time curves after administration of BH009 and Winthrop (docetaxel) Injection to advanced solid tumor patients. Referring to Figures 1 and 2, "R" stands for the commercial reference product (Winthrop injection) and "T" stands for test product (BH009).

### DETAILED DESCRIPTION

In one general aspect, provided herein is a pharmaceutical formulation containing docetaxel and human serum albumin. The formulation generally may be administered to a subject in need thereof by a parenteral route. The term "parenteral" refers to routes selected from subcutaneous ("SC"), intravenous ("IV"), intramuscular ("IM"), intrademal ("ID"), intraperitoneal ("IP") and the like. In one example, the formulation may be administered to the subject by intravenous infusion, such as from an infusion bag or bottle, as described herein.

In some embodiments, the formulation comprises docetaxel, human serum albumin, an acid, ethanol, and a liquid vehicle (e.g., parenterally acceptable vehicle). In some embodiments, the formulation comprises docetaxel, human serum albumin, citric acid, ethanol, and a liquid vehicle (e.g., parenterally acceptable vehicle). In some embodiments, the formulation contains in an infusion bag or bottle. In some embodiments, the formulation is prepared in an infusion bag or bottle.

In some embodiments, the pH value of the formulation is from about 4 to about 9.5. In some embodiments, the pH value of the formulation is from about 5 to about 9. In some embodiments, the pH value of the formulation is from about 6 to about 8. In some embodiments, the pH value of the formulation is from about 6.5 to about 7.5. In some embodiments, the pH value of the formulation is from about 4 to about 9. In some embodiments, the pH value of the formulation is from about 5 to about 8.5. In some embodiments, the pH value of the formulation is from about 6 to about 7.5.

In some embodiments, the formulation is substantially free from any surfactant. In some embodiments, the formulation is substantially free from polysorbate 80. In some embodiments, the formulation contains no more than about 1 ppm or about 1 ppb of a surfactant (e.g., polysorbate 80). In some embodiments, the formulation does not contain polysorbate 80. In some embodiments, the said pharmaceutical formulation does not comprise a lipid (e.g. soybean oil).

As used herein the term "docetaxel" refers to a compound that has the CAS No. 114977-28-5 and the following chemical structure: 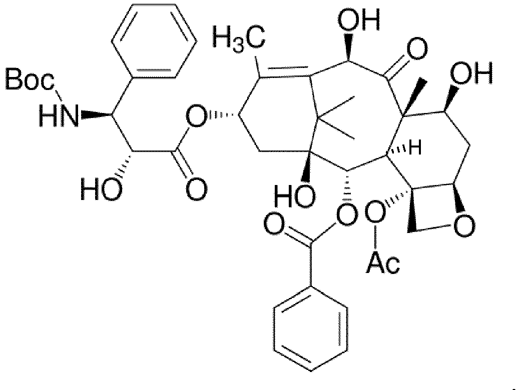 or a pharmaceutically acceptable salt thereof. Docetaxel is a white to almost-white powder. The compound is highly lipophilic and practically insoluble in water. Docetaxel is a microtubule inhibitor indicated for treating breast cancer, non-small cell lung cancer, hormone refractory prostate cancer, gastric adenocarcinoma, and squamous cell carcinoma of the head and neck cancer. The term "docetaxel" as used herein includes any of the pharmaceutically acceptable salts of the free base or free acid docetaxel compound (e.g., as in chemical structure depicted above, or any tautomer thereof). Generally, pharmaceutically acceptable salts are those that retain the desired biological activity of the subject compound and exhibit minimal undesired toxicological effects. These pharmaceutically acceptable salts may be prepared *in situ* during the final isolation and purification of the compound, or by separately reacting the purified compound in its free acid or free base form with a suitable base or acid, respectively. The salts may impart greater stability or solubility to the molecule thereby facilitating preparation of the formulation within the instant claims. Suitable example of pharmaceutically acceptable acids addition salts of docetaxel include bile acid addition salts, hydrochloride, hydrobromide, nitrate, methylnitrate, sulfate, bisulfate, sulfamate, phosphate, acetate, hydroxyacetate, phenyl acetate, propionate, butyrate, isobutyrate, valerate, maleate, hydroxymaleate, acrylate, fumarate, malate, tartrate, citrate, salicylate, *p*-aminosalicyclate, glycollate, lactate, heptanoate, phthalate, oxalate, succinate, benzoate, o-acetoxybenzoate, chlorobenzoate, methylbenzoate, dinitrobenzoate, hydroxybenzoate, methoxybenzoate, mandelate, tannate, formate, stearate, ascorbate, palmitate, oleate, pyruvate, pamoate, malonate, laurate, glutarate, glutamate, estolate, methanesulfonate (mesylate), ethanesulfonate (esylate), 2-hydroxyethanesulfonate, benzenesulfonate (besylate), p-aminobenzenesulfonate, p-toluenesulfonate (tosylate), napthalene-2-sulfonate, ethanedisulfonate, hydrogen bisulfide, bitartrate, gluconate, glucuronate, para-bromophenylsulfonate, carbonate, pyrosulfate, sulfite, bisulfite, monohydrogen phosphate, dihydrogen phosphate, metaphosphate, pyrophosphate, chloride, bromide, iodide, decanoate, caprylate, caprate, propiolate, suberate, sebacate, butyne-1,4-dioate, hexyne-1,6-dioate, terephthalate, sulfonate, xylenesulfonate, phenylpropionate, phenylbutyrate, β-hydroxybutyrate, glycolate, propanesulfonate, naphthalene-1-sulfonate, naphthalene-2-sulfonate and 2,5-dihydroxybenzoate. Suitable examples of pharmaceutically acceptable base addition salts include hydroxide of alkali metals including sodium, potassium, and lithium; hydroxides of alkaline earth metals such as calcium and magnesium; hydroxides of other metals, such as aluminum and zinc; ammonia, organic amines such as unsubstituted or hydroxyl-substituted mono-, di-, or tri-alkylamines, dicyclohexylamine; tributyl amine; pyridine; N-methyl, N-ethylamine; diethylamine; triethylamine; mono-, bis-, or tris-(2-OH-(C₁-C₆)-alkylamine), such as N,N-dimethyl-N-(2-hydroxyethyl)amine or tri-(2-hydroxyethyl)amine; N-methyl-D-glucamine; morpholine; thiomorpholine; piperidine; pyrrolidine; and amino acids such as arginine or lysine.

In some embodiments, the docetaxel may include 1, 2, or 3 equivalents of the water solvate. In some embodiments, the docetaxel is a solvate comprising three equivalents of water. In some embodiments, docetaxel is the docetaxel trihydrate, docetaxel monohydrate, or docetaxel anhydrous. In some embodiments, the docetaxel contains one equivalent of the acetone solvate. In some embodiments, the docetaxel can be any one of docetaxel solvates disclosed, for example, in WO2010091650 or US2012007167, the disclosures of which are incorporated herein by reference in their entirety. In some embodiments, docetaxel is crystalline. In some embodiments, docetaxel is any one of the crystalline forms disclosed, for example, in WO2012115402, US8410294, US20100197944, US20100099897, US8357811, US20100160653, or US20070142457, the disclosures of which are incorporated herein by reference in their entirety. In some embodiments, docetaxel in amorphous. In some embodiments, docetaxel is any one of the amorphous forms disclosed, for example, in WO2008102374, the disclosure of which is incorporated herein by reference in its entirety.

In some embodiments, concentration of docetaxel in the formulation is from about 0.05 mg/ml to about 1 mg/ml, from about 0.1 mg/ml to about 0.8 mg/ml, from about 0.05 mg/ml to about 0.75 mg/ml, from about 0.1 mg/ml to about 0.5 mg/ml, from about 0.1 mg/ml to about 0.35 mg/ml, from about 0.15 mg/ml to about 0.4 mg/ml, from about 0.2 mg/ml to about 0.3 mg/ml, from about 0.2 mg/ml to about 0.4 mg/ml, from about 0.2 mg/ml to about 0.35 mg/ml, from about 0.25 mg/ml to about 0.35 mg/ml, or from about 0.5 mg/ml to about 0.75 mg/ml. In some embodiments, the amount (or concentration) of docetaxel in the formulation is about 0.05 mg/ml, about 0.1 mg/ml, about 0.15 mg/ml, about 0.2 mg/ml, about 0.25 mg/ml, about 0.3 mg/ml, about 0.35 mg/ml, about 0.4 mg/ml, about 0.45 mg/ml, about 0.5 mg/ml, about 0.6 mg/ml, about 0.74 mg/ml, about 0.75 mg/ml, about 0.8 mg/ml, or about 1 mg/ml. In some embodiments, the formulation contains from about 10 mg to about 300 mg, from about 20 mg to about 200 mg, from about 50 mg to about 250 mg, from about 60 mg to about 240 mg, from about 100 mg to about 200 mg, from about 120 mg to about 200 mg, or from about 100 mg to about 175 mg of docetaxel. In some embodiments, the formulation contains about 20 mg, about 40 mg, about 80 mg, about 120 mg, about 150 mg, about 160 mg, about 175 mg, about 200 mg, or about 250 mg of docetaxel. In some embodiments, the formulation contains from about 50 mg/m² to about 150 mg/m² of docetaxel, e.g., based on the individual patient's body surface area ("BSA"). In some embodiments, the formulation contains a dosage about 50 mg/m², about 60 mg/m², about 75 mg/m², about 90 mg/m², about 100 mg/m², or about 120 mg/m² of docetaxel, such as based on the individual patient's body surface area ("BSA"). For example, when the patient's body surface area is 2.0 and the dosage of docetaxel is about 75mg/m², the formulation contains about 150 mg of docetaxel.

As used herein, the term "human serum albumin" refers to native and recombinant human serum albumin. In some embodiments, the human serum albumin is a native human serum albumin. In some embodiments, the human serum albumin is a recombinant human serum albumin.

Native human serum albumin and other plasma proteins can be precipitated from human plasma by varying the pH and adding ethanol, in what is known as the Cohn fractionation process (Cohn EJ et al., J. Am. Chem. Soc. 1946; 68:459-475). By controlling the pH and ethanol content, semi-purified fractions of plasma proteins can be produced. One of the last proteins to precipitate in the Cohn process is native human serum albumin. After precipitation, a wet paste of crude native human serum albumin is obtained. Subsequent bioprocessing steps (purification, filtration, pasteurization, etc.) can be used to produce a purified, stabilized form of native human serum albumin for commercial use (Lin JJ et al., Pharmaceutical Research 2000; 17:391-6). Recombinant human serum albumin is a highly purified animal-, virus-, and prion-free product as alternative to native human serum albumin, to which it is structurally equivalent (Bosse D et al., J. Clin. Pharmacol. 2005; 45:57-67). Recombinant human serum albumin has been produced by various hosts, both prokaryotic and eukaryotic (Chen Z et al., Biochimica et Biophysica Acta 2013; 1830:5515-5525).

Human serum albumin (HSA) is a highly soluble globular protein of Mᵣ 65K and consists of 585 amino acids. HSA is the most abundant protein in the plasma and accounts for 70-80% of the colloid osmotic pressure of human plasma. The amino acid sequence of HSA contains a total of 17 disulphide bridges, one free thiol (Cys 34), and a single tryptophan (Trp 214). Intravenous use of HSA solution has been indicated for the prevention and treatment of hypovolumic shock (*see, e.g.,* Tullis, JAMA, 237, 355-360, 460-463, (1977) and Houser et al., Surgery, Gynecology and Obstetrics, 150, 811-816 (1980)) and in conjunction with exchange transfusion in the treatment of neonatal hyperbilirubinemia (*see, e.g.,* Finlayson, Seminars in Thrombosis and Hemostasis, 6, 85-120, (1980)).

Human serum albumin used herein can also be human serum albumin solution. Suitable human serum albumin solution include, but are not limited to, commercially available solutions of human serum albumin for infusion. The commercially available solutions of human serum albumin for infusion comprise pharmaceutically acceptable excipient(s) such as sodium N-acetyltryptophan, sodium caprylate, sodium chloride, sodium bicarbonate, sodium hydroxide, or acetic acid, and the like or mixtures thereof. In some embodiments, human serum albumin solution can be prepared by diluting commercially available solutions of human serum albumin for infusion with a parenterally acceptable vehicle.

Alternatively, human serum albumin solution can be prepared by mixing human serum albumin powder in water along with other pharmaceutically acceptable excipient(s) as available in the commercially available albumin products.

In some embodiments, the human serum albumin is a commercially available solution of human serum albumin for infusion. In some embodiments, the human serum albumin solution.is a commercially available solution of human serum albumin for infusion. In some embodiments, the human serum albumin solution comprises a commercially available solution of human serum albumin for infusion. In some embodiments, a commercially available solution of human serum albumin for infusion is used as the source of the human serum albumin. In some embodiments, the solution of human serum albumin for infusion is 5% solution of human serum albumin (w/v). In some embodiments, the solution of human serum albumin for infusion is 20% solution of human serum albumin (w/v). In some embodiments, the solution of human serum albumin for infusion is 25% solution of human serum albumin (w/v). In some embodiments, the human serum albumin solution is an aqueous solution prepared by diluting a commercially available solution of human serum albumin for infusion.

In some embodiments, concentration of human serum albumin in the formulation is from about 0.1% to about 20% (w/v), from about 0.05% to about 10% (w/v), from about 0.05% to about 5% (w/v), from about 0.05% to about 3% (w/v), from about 0.02% to about 3% (w/v), from about 0.2% to about 10% (w/v), from about 0.5% to about 15% (w/v), from about 0.5% to about 5% (w/v), from about 1% to about 10% (w/v), or from about 1% to about 3% (w/v). In some embodiments, concentration of human serum albumin in the formulation is about 0.05% (w/v), about 0.1 % (w/v), about 0.25 % (w/v), about 0.5 % (w/v), about 1 % (w/v), about 2 % (w/v), about 3 % (w/v), about 5 % (w/v), about 10 % (w/v), about 15 % (w/v), or about 20 % (w/v). In some embodiments, the formulation contains from about 1 g to about 50 g, from about 2 g to about 40 g, from about 1 g to about 10 g, from about 2 g to about 20 g, from about 1 g to about 5 g, or from about 5 g to about 10 g of human serum albumin.

In some embodiments, weight ratio of human serum albumin to docetaxel in the formulation is from about 20:1 to about 200:1, or from about 20:1 to about 100:1. In some embodiments, weight ratio of human serum albumin to docetaxel in the formulation is about 20:1, about 30:1, about 40:1, about 50:1, about 75:1, about 90:1, about 95:1, about 100:1, or about 150:1.

In some embodiments, the amount of liquid vehicle (e.g., parenterally acceptable vehicle) in the formulation is from about 10 mL to about 2 L, from about 50 mL to about 1 L, from about 100 mL to about 1 L, from about 250 mL to about 1 L,from about 100 mL to about 500 mL, or from about 250 mL to about 500 mL. Suitable examples of liquid vehicles include water (e.g., sterile water), normal saline (*e.g.,* 0.9 wt.% NaCl aqueous solution), or a dextrose solution (e.g., 5 wt.% dextrose solution).
In some embodiments, the formulation is a clear (e.g., transparent) infusion solution, not a nanoparticle suspension. In some embodiments, the formulation is a clear solution. In some embodiments, the clear infusion solution stays clear free of precipitates for at least 1 hour, at least 2 hours, at least 3 hours, at least 4 hours, at least 6 hours, or at least 8 hours. In some embodiments, the clear infusion solution stays clear free of precipitates for at least 1 hour, at least 2 hours, at least 3 hours, at least 4 hours, or at least 6 hours, when the clear solution is kept at about 15-30 °C.In some embodiments, the clear infusion solution stays clear free of precipitates for at least 1 hour, at least 2 hours, at least 3 hours, at least 4 hours, or at least 6 hours, when the clear solution is kept at about 18-25 °C. In some embodiments, the clear solution stays clear free of precipitates for at least 2 hours, at least 4 hours, at least 6 hours, at least 8 hours, at least 12 hours,or at least 24 hours, when the clear solution is kept at about 2-8 °C. In some embodiments, the clear infusion solution stays clear free of precipitates for at least 1 hours, at least 2 hours, at least 4 hours, at least 6 hours, or at least 8 hours, when the clear solution is kept in ambient temperature.

In some embodiments, at least about 30%, at least about 40%, at least about 50%, at least about 60%, or at least about 70% docetaxel in the formulation (clear infusion solution) is free (unbound) docetaxel. Free (unbound) docetaxel means the fraction of docetaxel in the infusion composition (solution) that is not bound (e.g., non-covalently) to human serum albumin. In one embodiment, the amount of free (unbound) docetaxel in the parenteral infusion composition (solution) is measured by ultrafiltration through a 30-kDa membrane. Measured by Dynamic Light Scattering (DLS), the parenteral infusion composition (solution) has almost identical profile in DLS compared to the related human serum albumin saline solution. In some embodiments, 1 mL of the formulation (e.g., parenteral infusion composition) comprises no more than 50 particles that are greater than 10 µm in size, no more than 100 particles that are greater than 10 µm in size, no more than 150 particles that are greater than 10 µm in size, or no more than 200 particles that are greater than 10 µm in size. In some embodiments, 1ml of the parenteral infusion composition comprises no more than 5 particles that are greater than 25 µm in size, no more than 10 particles that are greater than 25 µm in size, no more than 5 particles that are greater than 15 µm in size, or no more than 20 particles that are greater than 25 µm in size.

In some embodiments, the formulation comprises an organic acid. Examples of organic acids include citric acid, acetic acid, formic acid, aspartic acid, glutamic acid, ascorbic acid, benzoic acid, tartaric acid, lactic acid, maleic acid, and succinic acid, or a pharmaceutically acceptable salt thereof. In some embodiments, the formulation comprises from about 0.001 mg/ml to about 2 mg/ml of citric acid, from about 0.005 mg/ml to about 1 mg/ml of citric acid, or from about 0.01 mg/ml to about 0.5 mg/ml of citric acid. Without being bound by any particular theory, it is believed that the organic acid (e.g., citric acid) is added to the formulation to create or maintain the desired pH of the formulation (e.g., infusion solution). In some embodiments, the pH value of the parenteral infusion composition is from about 4 to about 9.5, from about 5 to about 9, from about 6 to about 8, from about 6.5 to about 7.4, from about 4 to about 9, from about 5 to about 8.5, or from about 6 to about 7.5.

In some embodiments, the formulation comprises an organic solvent, or a combination of organic solvents. In some embodiments, the organic solvent is an alcohol. Suitable examples of alcohols include ethanol, propylene glycol, and polyethylene glycol 300. In some embodiments, the amount of ethanol in the parenteral infusion composition of docetaxel is no more than about 10% (v/v), about 5% (v/v), about 2% (v/v), about 1.75% (v/v), about 1.5% (v/v), or about 1.4% (v/v).

In some embodiments, the formulation (e.g., a parenteral infusion composition) is prepared from a first liquid composition and a second aqueous composition, as described further herein. In some embodiments, the formulation is prepared less than about 24 hours, less than about 12 hours, less than about 8 hours, less than about 6 hours, less than about 4 hours, or less than about 1 hour prior to being infused to a cancer patient. After the first liquid composition and second aqueous composition are mixed following the injection of the first liquid composition into the infusion bag or bottle comprising the second aqueous composition, a clear infusion solution free of precipitates comprising human serum albumin, docetaxel, citric acid, and ethanol in a parenterally acceptable vehicle is obtained.

In some embodiments, the present disclosure provides a pharmaceutical composition for infusion comprising docetaxel at a dose of about 75 mg/m² and human serum albumin, wherein the composition does not contain polysorbate 80. In some embodiments, the present disclosure provides a pharmaceutical composition for infusion comprising docetaxel at a dose of from about 50 mg to about 200 mg and human serum albumin, wherein the composition does not contain polysorbate 80. In some embodiments, the composition is prepared within 24 hours prior to being infused to said patient. In some embodiments, the present disclosure provides a pharmaceutical composition prepared from a first liquid first liquid composition and a second aqueous composition, as described further.

### Methods of making a pharmaceutical formulation

In some embodiments, the present disclosure provides a method of making a formulation (e.g., pharmaceutical composition for infusion) comprising docetaxel and human serum albumin, the method comprising mixing (a) a first liquid composition comprising docetaxel and ethanol; and (b) a second aqueous composition comprising human serum albumin and a parenterally acceptable vehicle. In some embodiments, the first liquid composition and the second aqueous composition do not comprise polysorbate 80, a surfactant, or a lipid (e.g., soybean oil). In some embodiments, the two compositions are mixed in less than about 24 hours, about 8 hours, about 4 hours, or about 1 hour prior to being infused or administered to patients. In some embodiments, the mixing is done in an infusion bag or bottle. For example, the second aqueous composition is contained in an infusion bag or bottle, and the first liquid composition is injected into an infusion bag or bottle which contains the second aqueous composition. In some embodiments, a syringe is used to inject the first liquid composition into an infusion bag or bottle which contains the second aqueous composition.

In some embodiments, the first liquid composition comprises docetaxel and ethanol. In some embodiments, the first liquid composition comprises docetaxel, ethanol, and other organic solvents (e.g. propylene glycol, polyethylene glycol 300, etc.). In some embodiments, the first liquid composition further comprises an acid (e.g., citric acid). Preferably the organic acid is selected in the group consisting of citric acid, acetic acid, formic acid, ascorbic acid, benzoic acid, tartaric acid, lactic acid, maleic acid, and succinic acid, or a pharmaceutically acceptable salt thereof. In some embodiments, the first liquid composition is an ethanol solution containing docetaxel. In some embodiments, the weight ratio of docetaxel and citric acid, in the first liquid composition is from about 5000:1 to about 1:1, from about 2000:1 to about 1:1, from about 1000:1 to about 1:1, from about 500:1 to about 1:1, from about 5000:1 to about 5:1, from about 2000:1 to about 5:1, from about 1000:1 to about 5:1, from about 500:1 to about 5:1, from about 5000:1 to about 10:1, from about 2000:1 to about 10:1, from about 1000:1 to about 10:1, from about 500:1 to about 10:1, from about 2000:1 to about 100:1, from about 1000:1 to about 100:1, from about 500:1 to about 100:1, or from about 500:1 to about 200:1. In some embodiments, the weight ratio of docetaxel and citric acid, in the first liquid composition is about 1000:1, about 500:1, about 200:1, or about 100:1. In some embodiments, the concentration of citric acid in the first liquid composition is from about 0.005 mg/ml to about 10 mg/ml, from about 0.01 mg/ml to about 2 mg/ml, from about 0.01 mg/ml to about 1 mg/ml, from about 0.02 mg/ml to about 0.5 mg/ml, from about 0.02 mg/ml to about 0.2 mg/ml. In some embodiments, the first liquid composition is prepared by dissolving docetaxel (and/or citric acid) in an alcohol (e.g., ethanol and/or propylene glycol). In some embodiments, concentration of docetaxel in the alcohol (e.g., ethanol and/or propylene glycol) is from about 1 mg/ml to about 200 mg/ml, from about 5 mg/ml to about 100 mg/ml, from about 5 mg/ml to about 40 mg/ml, from about 10 mg/ml to about 50 mg/ml, from about 10 mg/ml to about 20 mg/ml, from about 10 mg/ml to about 30 mg/ml. In some embodiments, concentration of docetaxel in the alcohol (e.g., ethanol and/or propylene glycol) is about 10 mg/ml, about 15 mg/ml, about 20 mg/ml, or about 30 mg/ml. In some embodiments, the amount of docetaxel in the first liquid composition is from about 10 mg to about 300 mg, or from about 20 mg to about 200 mg. In some embodiments, amount of docetaxel in the first liquid composition is about 20 mg, about 40 mg, about 60 mg, about 80 mg, about 100 mg, about 120 mg, or about 160 mg. In some embodiments, amount of ethanol in the first liquid composition is rom about 0.5 ml to about 50 ml, from about 1 ml to about 20 ml, from about 1 ml to about 10 ml, or from about 3 ml to about 6 ml.

Generally, the first liquid composition is an injectable composition (e.g., it is made or adapted for injecting into an infusion bag or bottle containing the aqueous composition comprising human serum albumin). In some embodiments, the first liquid composition is an injectable pharmaceutical formulation. The concentration of docetaxel in the injectable pharmaceutical composition (pharmaceutical formulation) may be from about 5 mg/ml to about 40 mg/ml, or from about 10 mg/ml to about 20 mg/ml. In some embodiments, concentration of docetaxel the injectable pharmaceutical composition (pharmaceutical formulation) is about 10 mg/ml, about 15mg/ml, or about 20 mg/ml. In some embodiments, pH of the injectable pharmaceutical composition (fist liquid composition) is from about 3 to about 9, from about 3 to about 8, from about 3 to about 7, from about 3 to about 6.5, from about 3.5 to about 6.5, from about 3.5 to about 6, or from about 3.5 to about 5.5, or from about 4 to about 5. In one example, to measure the pH value of the injectable pharmaceutical dosage form, the injectable pharmaceutical dosage form is mixed with normal saline in 1:1 ratio (v/v) to obtain an aqueous solution, and then pH value of the aqueous solution is tested. In some embodiments, an injectable pharmaceutical dosage form comprising the first liquid composition comprises about 20 mg, about 40 mg, about 80 mg, about 120 mg, or about 160 mg of docetaxel, citric acid and ethanol, wherein concentration of docetaxel in the injectable pharmaceutical dosage form is from about 5 mg/ml to about 40 mg/ml (e.g., about 20 mg/ml or about 10 mg/ml), and pH of the composition is from about 3 to about 6.5, or from about 3.5 to about 5.5.

In some embodiments, the second aqueous composition is prepared by adding a human serum albumin solution into a parenterally acceptable vehicle. For example, the second aqueous composition is prepared by adding a human serum albumin solution into an infusion bag or bottle which contains a parenterally acceptable vehicle (e.g., a normal saline solution or a dextrose solution). In some embodiments, the second aqueous composition is prepared by adding about 20%, about 25%, or about 5% solution of human serum albumin for infusion (w/v) into a normal saline solution or dextrose solution. In some embodiments, the concentration of human serum albumin in the second aqueous composition is from about 0.1% to about 20% (w/v), from about 0.5% to about 10% (w/v), from about 0.5% to about 15% (w/v), from about 0.5% to about 5% (w/v), from about 1% to about 10% (w/v), or from about 1% to about 3% (w/v). In some embodiments, the second aqueous composition comprises from about 1 g to about 50 g of human serum albumin, from about 1g to about 20 g, from about 2g to about 10 g, or from about 2 g to about 20 g of human serum albumin. In some embodiments, the volume of the second aqueous composition is from about 100 ml to about 1L. In some embodiments, the volume of the second aqueous composition is from about 250 ml to about 1L. In some embodiments, the volume of the second aqueous composition is from about 250 ml to about 500 ml.

Generally, the injection of the first liquid composition into the infusion bag or bottle containing the second aqueous composition is rapid. In some embodiments, the injection time is no more than 60 seconds, no more than 30 seconds, no more than 15 seconds, no more than 10 seconds, no more than 5 seconds or no more than 3 seconds. Generally, in the process of injecting the first liquid composition into the infusion bag or bottle containing the second aqueous composition, mixing or agitation (e.g., deliberate stirring) is not needed. After the injection of the first liquid composition into the infusion bag or bottle is completed, the first liquid composition and second aqueous composition are mixed well (e.g., the bag containing the composition is gently inverted by hand) to obtain a clear infusion solution free of precipitates. In some embodiments, the infusion bag or bottle containing the second aqueous composition is held still in the injection process. In some embodiments, the first liquid composition is injected underneath the liquid surface of the second aqueous composition in the injection process. In some embodiments, after the injection is completed, the infusion bag or bottle is gently inverted repeatedly to mix well of the first liquid composition and second aqueous composition. By simply inverting the infusion bag or bottle gently (e.g. about 5-30 times), a clear infusion solution free of precipitates may be obtained. The mixing time can be, for example, from about 5 seconds to about 10 minutes, from about 5 seconds to about 3 minutes, from about 5 seconds to about 2 minutes, or from about 0.1 minute to about 1 minute. After the first liquid composition and second aqueous composition are mixed well followed the injection of the first liquid composition into the infusion bag or bottle comprising the second aqueous composition, a clear infusion solution free of precipitates comprising human serum albumin, docetaxel, ethanol, and citric acid in a parenterally acceptable vehicle is obtained. In some embodiments, in the injection process, no agitation (e.g., stirring) is needed to mix the compositions. In some embodiments, the injection of the first liquid composition into the infusion bag or bottle containing the second aqueous composition is done in the temperature from about 15 °C to about 30 °C, from about 15 °C to about 25 °C, or from about 20 °C to about 25 °C. In some embodiments, the injection of the first liquid composition into the infusion bag or bottle containing the second aqueous composition is done in ambient temperature.

In some embodiments, the present disclosure provides methods of preparing a clear (e.g., free from precipitates) parenteral infusion composition of docetaxel as described herein. An embodiment of the method is as follows:
1) dissolve docetaxel and an acid (e.g., citric acid) in ethanol or a mixed solvent comprising ethanol (e.g., a mixed solvent of ethanol and PEG300, or ethanol and PEG400) to prepare a first liquid composition;
2) add a human serum albumin solution (e.g., a commercially and clinically useful 20%, 25%, or 5% solution of human serum albumin for infusion (w/v)) into an infusion bag or bottle containing a parenterally acceptable vehicle (e.g., a normal saline solution or dextrose solution) to prepare the second aqueous composition;
3) inject the first liquid composition into the infusion bag or bottle containing the second aqueous composition; in the injection process, the infusion bag or bottle is held still and mixing or agitation is not need;
4) after the injection of the first liquid composition into the infusion bag or bottle is completed, the first liquid composition and second aqueous composition are mixed well (e.g., the infusion bag or bottle containing the composition is gently inverted by hand) to obtain a clear infusion solution free of precipitates.

One embodiment of the method is as follows, e.g., the method comprises:
1) dissolving docetaxel and an acid (e.g., citric acid) in ethanol or a mixed solvent comprising ethanol (e.g., a mixed solvent of ethanol and PEG300, or ethanol and PEG400) to prepare the first liquid composition (e.g., the method comprises obtaining a first liquid composition comprising a solution of docetaxel and an acid in ethanol or a mixed solvent comprising ethanol);
2) adding a human serum albumin solution (e.g., a clinically used 20%, 25%, or 5% solution of human serum albumin for infusion (w/v)) into an infusion bag or bottle containing a parenterally acceptable vehicle (e.g., a normal saline solution or dextrose solution) to prepare the second aqueous composition (e.g., the method comprises obtaining an infusion bag or bottle comprising a second aqueous composition comprising a solution of HSA and a parenterally acceptable vehicle);
3) injecting the first liquid composition into the infusion bag or bottle containing the second aqueous composition; in the injection process, the infusion bag or bottle is held still and mixing or agitation is not need (e.g., the method comprises combining the first liquid composition and the second aqueous composition by injecting the first liquid composition into the infusion bag of bottle comprising the second aqueous composition without mixing the compositions and/or without agitation of the infusion bag or bottle (e.g., by holding still the infusion bag or bottle)); and
4) after the injection of the first liquid composition into the infusion bag or bottle is completed, mixing well the first liquid composition and second aqueous composition (e.g., gently inverting by hand the infusion bag or bottle containing the composition) to obtain a clear infusion solution free of precipitates The first step and the second step of the preparation could be done in parallel or any different order. Once the mixing is completed, the formulation thus obtained may be then administered to a cancer patient at the required dosage level, for example, at a dosage from about 50 mg/m² to about 150 mg/m² of docetaxel based on the individual patient's body surface area ("BSA"). In some embodiments, the formulation may be administered at about 50 mg/m², about 60 mg/m², about 75 mg/m², about 90 mg/m², about 100 mg/m², or about 120 mg/m² of docetaxel.

### Methods of treating cancer

The present disclosure also provides a method of treating cancer, the method comprising administering to a subject in need thereof a therapeutically effective amount of any composition or formulation described herein. In some embodiments, the method comprises parenterally administering to a subject in need thereof of a therapeutically effective amount of a liquid infusion formulation of the present disclosure. In some embodiments, the formulation may contain, or may be configured to be administered at a dose of about 50 mg/m², about 60 mg/m², about 75 mg/m², about 90 mg/m², about 100 mg/m², or about 120 mg/m² of docetaxel, based on the BSA of the patient.

As used herein, the term "body surface area," or "BSA," refers to measured or calculated surface area of a human body. This measure is generally used as an indicator of metabolic mass than body weight. The chart below shows body surface area for typical heights and weights. Calculations were made using the DuBois & DuBois formula: kg.⁴²⁵ × cm.⁷²⁵ × 0.007184. Weight is the horizontal axis, first in pounds, then in kilograms. Height is the vertical axis, first in inches, then in centimeters. Results are body surface areas in square meters. Exemplary BSA are provided in the chart below.

| **Lbs/ Kg** | **100/ 45** | **110/ 50** | **120/ 54** | **130/ 59** | **140/ 63** | **150/ 68** | **160/ 72** | **170/ 77** | **180/ 81** | **190/ 86** | **200/ 90** | **210/ 95** | **220/ 99** | **230/ 104** | **240/ 108** | **250/ 113** | **260/ 117** | **270/ 122** |
|---|---|---|---|---|---|---|---|---|---|---|---|---|---|---|---|---|---|---|
| **In/Cm** | | | | | | | | | | | | | | | | | | |
| **60/ 152.4** | 1.4 | 1.4 | 1.5 | 1.6 | 1.6 | 1.7 | 1.7 | 1.7 | 1.8 | 1.8 | 1.9 | 1.9 | 1.9 | 1.97 | 2 | 2.04 | 2.1 | 2.11 |
| **61/ 154.9** | 1.4 | 1.5 | 1.5 | 1.6 | 1.6 | 1.7 | 1.7 | 1.8 | 1.8 | 1.8 | 1.9 | 1.9 | 2 | 2.00. | 2 | 2.07 | 2.1 | 2.14 |
| **62/ 157.5** | 1.4 | 1.5 | 1.5 | 1.6 | 1.6 | 1.7 | 1.7 | 1.8 | 1.8 | 1.9 | 1.9 | 1.9 | 2 | 2.02 | 2.1 | 2.09 | 2.1 | 2.16 |
| **63/ 160** | 1.4 | 1.5 | 1.6 | 1.6 | 1.7 | 1.7 | 1.8 | 1.8 | 1.8 | 1.9 | 1.9 | 2 | 2 | 2.04 | 2.1 | 2.12 | 2.2 | 2.19 |
| **64/ 162.6** | 1.5 | 1.5 | 1.6 | 1.6 | 1.7 | 1.7 | 1.8 | 1.8 | 1.9 | 1.9 | 2 | 2 | 2 | 2.07 | 2.1 | 2.14 | 2.2 | 2.21 |
| **65/ 165.1** | 1.5 | 1.5 | 1.6 | 1.6 | 1.7 | 1.7 | 1.8 | 1.8 | 1.9 | 1.9 | 2 | 2 | 2.1 | 2.09 | 2.1 | 2.17 | 2.2 | 2.24 |
| **66/ 167.6** | 1.5 | 1.6 | 1.6 | 1.7 | 1.7 | 1.8 | 1.8 | 1.9 | 1.9 | 2 | 2 | 2 | 2.1 | 2.11 | 2.2 | 2.19 | 2.2 | 2.26 |
| **67/ 170.2** | 1.5 | 1.6 | 1.6 | 1.7 | 1.7 | 1.8 | 1.8 | 1.9 | 1.9 | 2 | 2 | 2.1 | 2.1 | 2.14 | 2.2 | 2.21 | 2.3 | 2.29 |
| **68/ 172.7** | 1.5 | 1.6 | 1.6 | 1.7 | 1.8 | 1.8 | 1.9 | 1.9 | 2 | 2 | 2 | 2.1 | 2.1 | 2.16 | 2.2 | 2.24 | 2.3 | 2.31 |
| **69/ 175.3** | 1.5 | 1.6 | 1.7 | 1.7 | 1.8 | 1.8 | 1.9 | 1.9 | 2 | 2 | 2.1 | 2.1 | 2.1 | 2.18 | 2.2 | 2.26 | 2.3 | 2.34 |
| **70/ 177.8** | 1.6 | 1.6 | 1.7 | 1.7 | 1.8 | 1.8 | 1.9 | 1.9 | 2 | 2 | 2.1 | 2.1 | 2.2 | 2.21 | 2.3 | 2.29 | 2.3 | 2.36 |
| **71/ 180.3** | 1.6 | 1.6 | 1.7 | 1.8 | 1.8 | 1.9 | 1.9 | 2 | 2 | 2.1 | 2.1 | 2.2 | 2.2 | 2.23 | 2.3 | 2.31 | 2.4 | 2.39 |
| **72/ 182.3** | 1.6 | 1.6 | 1.7 | 1.8 | 1.8 | 1.9 | 1.9 | 2 | 2 | 2.1 | 2.1 | 2.2 | 2.2 | 2.25 | 2.3 | 2.33 | 2.4 | 2.41 |
| **73/ 185.4** | 1.6 | 1.7 | 1.7 | 1.8 | 1.8 | 1.9 | 2 | 2 | 2.1 | 2.1 | 2.1 | 2.2 | 2.2 | 2.27 | 2.3 | 2.36 | 2.4 | 2.44 |
| **74/ 188** | 1.6 | 1.7 | 1.7 | 1.8 | 1.9 | 1.9 | 2 | 2 | 2.1 | 2.1 | 2.2 | 2.2 | 2.3 | 2.3 | 2.3 | 2.38 | 2.4 | 2.46 |
| **75/ 190.5** | 1.6 | 1.7 | 1.8 | 1.8 | 1.9 | 1.9 | 2 | 2 | 2.1 | 2.1 | 2.2 | 2.2 | 2.3 | 2.32 | 2.4 | 2.4 | 2.5 | 2.48 |
| **76/ 193** | 1.6 | 1.7 | 1.8 | 1.8 | 1.9 | 2 | 2 | 2.1 | 2.1 | 2.2 | 2.2 | 2.3 | 2.3 | 2.34 | 2.4 | 2.43 | 2.5 | 2.51 |
| **77/ 195.6** | 1.7 | 1.7 | 1.8 | 1.9 | 1.9 | 2 | 2 | 2.1 | 2.1 | 2.2 | 2.2 | 2.3 | 2.3 | 2.36 | 2.4 | 2.45 | 2.5 | 2.53 |
| **78/ 198** | 1.7 | 1.7 | 1.8 | 1.9 | 1.9 | 2 | 2.1 | 2.1 | 2.2 | 2.2 | 2.3 | 2.3 | 2.3 | 2.39 | 2.4 | 2.47 | 2.5 | 2.56 |

In one example, an average BSA for cancer patients is about 1.73 m².

In some embodiments, when the formulation herein is infused to the patient, the infusion bag of bottle, after injection of the predetermined amount of the first liquid composition, contains the required dose amount, for example, about 75 mg/m² of docetaxel. In some embodiments, multiple infusion bags or bottles are used to prepare the infusion formulation which contains the required dose amount for one infusion to the patient. In one example, for a cancer patient with BSA of about 2 m², the dose of docetaxel in the infusion bag is about 150 mg. In some embodiments, the infusion time is from about 1 hour to about 2 hours. In some embodiments, the infusion time is about 1 hour. The liquid infusion flow rate may then be determined and/or adjusted by the desired infusion time (e.g., 1 hour). In other embodiments, one infusion bag or multiple infusion bags (e.g., 2 infusion bags) may contain an amount of docetaxel in the required dosage. In this case, the required dosage, such as a dose of about 75 mg/m², can be administered to the patient by adjusting the infusion rate (drip rate) in the desired infusion time (e.g., 1 hour). In some embodiments, the concentration of docetaxel in the infusion bag is from about 0.1 mg/ml to about 0.5 mg/ml. In some embodiments, the concentration of docetaxel in the infusion bag is from about 0.15 mg/ml to about 0.4 mg/ml. In some embodiments, the concentration of docetaxel in the infusion bag is about 0.2 mg/ml, about 0.25 mg/ml, about 0.30 mg/ml, or about 0.35 mg/ml. The volume amount of infusion solution may be determined based on the concentration of docetaxel in the infusion bag. For example, if the required dosage is 120 mg docetaxel for one infusion (treatment) and the concentration of docetaxel in the infusion bag is 0.25 mg/ml, the total volume of infusion solution in the infusion bag will be 480 ml. The desired amount of infusion solution to the patient may be contained in one infusion bag or multiple infusion bags (e.g., 2 infusion bags) based on the volume of the infusion solution and size of the infusion bag. For example, the infusion bag may contain about 450 mL of infusion solution containing about 90 mg of docetaxel at a concentration of about 0.20 mg/mL. In another example, two infusion bags in total may contain about 600 mL of infusion solution containing about 150 mg of docetaxel at a concentration of about 0.25 mg/mL.

In some embodiments, cancer is selected from sarcoma, angiosarcoma, fibrosarcoma, rhabdomyosarcoma, liposarcoma, myxoma, rhabdomyoma, fibroma, lipoma, teratoma, non-small cell lung cancer (NSCLC), bronchogenic carcinoma squamous cell, undifferentiated small cell, undifferentiated large cell, adenocarcinoma, alveolar bronchiolar carcinoma, bronchial adenoma, sarcoma, lymphoma, chondromatous hamartoma, mesothelioma, gastrointestinal cancer, cancer of the esophagus, squamous cell carcinoma, adenocarcinoma, leiomyosarcoma, lymphoma, cancer of the stomach, carcinoma, lymphoma, leiomyosarcoma, cancer of the pancreas, ductal adenocarcinoma, insulinoma, glucagonoma, gastrinoma, carcinoid tumor, vipoma, cancer of the small bowel, adenocarcinoma, lymphoma, carcinoid tumors, Kaposi's sarcoma, leiomyoma, hemangioma, lipoma, neurofibroma, fibroma, cancer of the large bowel or colon, tubular adenoma, villous adenoma, hamartoma, leiomyoma, genitourinary tract cancer , cancer of the kidney adenocarcinoma, Wilm's tumor (nephroblastoma), lymphoma, leukemia, cancer of the bladder, cancer of the urethra, squamous cell carcinoma, transitional cell carcinoma, cancer of the prostate, cancer of the testis, seminoma, teratoma, embryonal carcinoma, teratocarcinoma, choriocarcinoma, sarcoma, interstitial cell carcinoma, fibroma, fibroadenoma, adenomatoid tumors, lipoma, liver cancer, hepatoma hepatocellular carcinoma, cholangiocarcinoma, hepatoblastoma, angiosarcoma, hepatocellular adenoma, hemangioma, bone cancer, osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor, chordoma, osteochrondroma (osteocartilaginous exostoses), benign chondroma, chondroblastoma, chondromyxofibroma, osteoid osteoma giant cell tumor, nervous system cancer, cancer of the skull, osteoma, hemangioma, granuloma, xanthoma, osteitis deformans, cancer of the meninges meningioma, meningiosarcoma, gliomatosis, cancer of the brain, astrocytoma, medulloblastoma, glioma, ependymoma, germinoma (pinealoma), glioblastoma multiforme, oligodendroglioma, schwannoma, retinoblastoma, congenital tumors, cancer of the spinal cord, neurofibroma, meningioma, glioma, sarcoma, gynecological cancer, cancer of the uterus, endometrial carcinoma, cancer of the cervix, cervical carcinoma, pre tumor cervical dysplasia, cancer of the ovaries, ovarian carcinoma, serous cystadenocarcinoma, mucinous cystadenocarcinoma, unclassified carcinoma, granulosa-theca cell tumor, Sertoli Leydig cell tumor, dysgerminoma, malignant teratoma, cancer of the vulva, squamous cell carcinoma, intraepithelial carcinoma, adenocarcinoma, fibrosarcoma, melanoma, cancer of the vagina, clear cell carcinoma, squamous cell carcinoma, botryoid sarcoma, embryonal rhabdomyosarcoma, cancer of the fallopian tubes, hematologic cancer, cancer of the blood, acute myeloid leukemia (AML), chronic myeloid leukemia (CML), acute lymphoblastic leukemia (ALL), chronic lymphoblastic leukemia, chronic lymphocytic leukemia, myeloproliferative diseases, multiple myeloma, myelodysplastic syndrome, Hodgkin's lymphoma, non-Hodgkin's lymphoma (malignant lymphoma), Waldenstrom's macroglobulinemia, skin cancer, malignant melanoma, basal cell carcinoma, squamous cell carcinoma, Kaposi's sarcoma, moles dysplastic nevi, lipoma, angioma, dermatofibroma, keloids, psoriasis, adrenal gland cancer, and neuroblastoma.

In some embodiments, the cancer is a solid tumor cancer. Some examples of solid tumors include: breast cancer, prostate cancer, lung cancer, liver cancer, pancreatic cancer, and melanoma. In some embodiments, the cancer is selected from breast cancer, non-small cell lung cancer, prostate cancer (such as castration-resistant prostate cancer), gastric cancer (such as adenocarcinoma), and head and neck cancer (such as squamous cell carcinoma of the head and neck).

In some embodiments, the method of treating cancer of this disclosure include administering the docetaxel in combination with an additional anti-cancer therapeutic. Suitable examples of the additional therapeutics include doxorubicin, cyclophosphamide, cisplatin, prednisone, and fluorouracil, or a combination thereof. Any of these additional therapeutic agents can be administered to the patient either consecutively (before or after) or simultaneously with the docetaxel formulation of the instant claims. The additional therapeutic agent can be administered at a dose from about 50 mg/m² to about 750 mg/m² depending on the patient's exact diagnosis and the recommendation of the treating physician.

In some embodiment, the present disclosure provides a method of treating a cancer in the subject (e.g., breast cancer, non-small cell lung cancer, prostate cancer, gastric cancer, and head and neck cancer), the method comprising:
i) mixing a first liquid formulation as described herein with a second aqueous composition as described herein to obtain a formulation comprising docetaxel for infusion; and
ii) administering the formulation obtained in step (i) to the subject in need thereof by infusion at the required dose of docetaxel (e.g., e.g., about 50 mg/m², about 60 mg/m², about 75 mg/m², about 90 mg/m², about 100 mg/m², or about 120 mg/m² of docetaxel, based on the BSA of the patient).

In some embodiment, the present disclosure provides a method of treating a cancer in the subject (e.g., breast cancer, non-small cell lung cancer, prostate cancer, gastric cancer, and head and neck cancer), the method comprising:
i) mixing a first liquid formulation as described herein with a second aqueous composition as described herein to obtain a formulation for infusion comprising the required dose of docetaxel (e.g., e.g., about 50 mg/m², about 60 mg/m², about 75 mg/m², about 90 mg/m², about 100 mg/m², or about 120 mg/m² of docetaxel, based on the BSA of the patient); and
ii) administering the formulation obtained in step (i) to the subject in need thereof by infusion at the required dose of docetaxel.

In some embodiment, the formulation for infusion comprising the required dose of docetaxel (e.g., about 50 mg/m², about 60 mg/m², about 75 mg/m², about 90 mg/m², about 100 mg/m², or about 120 mg/m² of docetaxel, based on the BSA of the patient) can be prepared in multiple infusion bags or bottles (e.g., in 2 infusion bags or bottles).

### Kits

Also, provided herein is a kit for the preparation of the formulation for infusion described herein, comprising: a first container containing a composition comprising docetaxel; and a second container containing a composition comprising human serum albumin. In some embodiments, the kit comprises a first container containing a liquid composition comprising docetaxel, and a second container containing a liquid composition comprising human serum albumin. In some embodiments, the kit comprises a first container containing a liquid composition comprising docetaxel and ethanol, and a second container containing a liquid composition comprising human serum albumin. In some embodiments, the kit comprises a first container containing a liquid composition comprising docetaxel, citric acid, and ethanol, and a second container containing a liquid composition comprising human serum albumin. In some embodiments, the kit comprises a first container containing a liquid composition comprising docetaxel, citric acid, and dehydrated ethanol, and a second container containing a liquid composition comprising human serum albumin. In some embodiments, the kit comprises a first container containing a liquid composition comprising docetaxel, citric acid, and ethanol, and a second container containing a solution of human serum albumin (e.g. 20%, 25%, or 5% (w/v)). In some embodiments, both the liquid compositions in the first container and the second container are sterile solutions.

In some embodiments, ethanol contained in the first container is dehydrated ethanol. In some embodiments, the first container contains a liquid composition comprising docetaxel and ethanol. In some embodiments, the first container contains a liquid composition comprising docetaxel, an acid, and ethanol. In some embodiments, the first container contains a liquid composition comprising docetaxel, an organic acid, and ethanol. In some embodiments, the first container contains a liquid composition comprising docetaxel, ethanol, and other organic solvents (e.g., an alcohol such as propylene glycol, or polyethylene glycol 300, etc.). In some embodiments, the first container contains a liquid composition comprising docetaxel, citric acid, and ethanol. In some embodiments, the first container contains an ethanol solution containing docetaxel. In some embodiments, the first container contains an ethanol solution containing docetaxel and citric acid. In some embodiments, the concentration of citric acid in the first container is from about 0.005 mg/ml to about 10 mg/ml. In some embodiments, the concentration of citric acid in the first container is from about 0.01 mg/ml to about 2 mg/ml. In some embodiments, the concentration of citric acid in the first container is from about 0.01 mg/ml to about 1 mg/ml. In some embodiments, the concentration of citric acid in the first container is from about 0.02 mg/ml to about 0.5 mg/ml. In some embodiments, the concentration of citric acid in the first container is from about 0.02 mg/ml to about 0.2 mg/ml.

In some embodiments, the amount of docetaxel contained in the first container is from about 10 mg to about 300 mg of docetaxel. In some embodiments, the amount of docetaxel contained in the first container is from about 20 mg to about 200 mg of docetaxel. In some embodiments, the amount of docetaxel contained in the first container is about 20 mg, about 40 mg, about 60 mg, about 80 mg, about 100 mg, about 120 mg, or about 160 mg of docetaxel. In some embodiments, the amount of ethanol contained in the first container is from about 0.5 ml to about 50 ml of ethanol. In some embodiments, the amount of ethanol contained in the first container is from about 1 ml to about 20 ml of ethanol. In some embodiments, the amount of ethanol contained in the first container is from about 1 ml to about 10 ml of ethanol. In some embodiments, the amount of ethanol contained in the first container is from about 1 ml to about 5 ml of ethanol. In some embodiments, the amount of ethanol contained in the first container is from about 5 ml to about 10ml of ethanol. In some embodiments, the concentration of docetaxel in the first container is from about 5 mg/ml to about 100 mg/ml. In some embodiments, the concentration of docetaxel in the first container is from about 10 mg/ml to about 40 mg/ml. In some embodiments, the concentration of docetaxel in the first container is about 20 mg/ml.

In some embodiments, the first container is an injectable pharmaceutical product comprising the first liquid composition described herein.In some embodiments, the first container is an injectable pharmaceutical product comprising about 0.5 ml to about 30 ml of the first liquid composition described herein. In some embodiments, the first container is an injectable pharmaceutical product comprising about 1 ml to about 20 ml of the first liquid composition described herein.In some embodiments, the first container is an injectable pharmaceutical product comprising about 1 ml to about 10 ml of the first liquid composition described herein. In some embodiments, the first container is an injectable pharmaceutical product comprising about 1 ml about 2 ml,about 4 ml, about 6 ml and about 8 ml of the first liquid composition described herein. In some embodiments, the first container comprises about 80mg docetaxel. In some embodiments, the first container comprises about 80mg docetaxel and about 4ml ethanol.

In some embodiments, the second container contains a liquid composition comprising human serum albumin. In some embodiments, the second container contains a human serum albumin solution. In some embodiments, the second container contains a human serum albumin solution with the concentration of human serum albumin in the solution from about 1% to about 25% (w/v). In some embodiments, the second container contains a human serum albumin solution with the concentration of human serum albumin in the solution from about 5% to about 25% (w/v). In some embodiments, the second container contains a solution of human serum albumin for infusion. In some embodiments, the second container contains a 20% solution of human serum albumin for infusion (w/v). In some embodiments, the second container contains a 25% solution of human serum albumin for infusion (w/v). In some embodiments, the second container contains a 5% solution of human serum albumin for infusion (w/v). In some embodiments, the second container contains a liquid composition comprising from about 1 g to about 50 g of human serum albumin. In some embodiments, the second container contains a liquid composition comprising from about 1 g to about 30 g of human serum albumin. In some embodiments, the second container contains a liquid composition comprising from about 1 g to about 20 g of human serum albumin. In some embodiments, the second container contains a liquid composition comprising from about 1 g to about 10 g of human serum albumin. In some embodiments, the second container contains a liquid composition comprising from about 2 g to about 6 g of human serum albumin. In some embodiments, the second container contains a liquid composition comprising about 2 g, about 4 g, about 6 g, about 8 g, about 10 g, about 12 g, or about 16 g of human serum albumin.

In some embodiments, the first container comprises about 80mg docetaxel. In some embodiments, the first container comprises about 4ml ethanol. In some embodiments, the first container comprises about 80mg docetaxel and about 4ml ethanol.

In some embodiments, the kit comprises the first container comprising about 80mg docetaxel and the second container comprising from about 2g to about 10g of human serum albumin. In some embodiments, the kit comprises the first container comprising about 80mg docetaxel and the second container comprising about 10g of human serum albumin.

In some embodiments, the desired amount of first liquid composition is taken out from one or multiple first containers by a syringe and injected into the infusion bag or bottle containing the second aqueous composition to prepare the formulation for infusion described herein.

In some embodiments, the present disclosure provides methods for preparing an infusion docetaxel composition for the treatment of cancer (e.g., breast cancer, non-small cell lung cancer, prostate cancer, gastric cancer, head and neck cancer) in a subject. An embodiment of the preparation method is as follows:
1) Withdrawing the desired volume of human serum albumin solution (e.g., a commercially available and clinically used 20%, 25%, or 5% (w/v)solutions of human serum albumin for infusion ) from one or more secondary containers using a syringe, the withdrawn solution is then injected into an infusion bag or bottle containing a corresponding desired volume of a parenterally acceptable vehicle (e.g., a normal saline solution or dextrose solution) a second aqueous composition is obtained after mixing;
2) Withdrawing the first liquid composition containing the desired dose of docetaxel from one or more first containers using a syringe;
3) Injecting the first liquid composition withdrawn from the aforementioned 2) into the infusion bag or bottle containing the second aqueous composition described in 1);
4) Upon completion of injection the first liquid composition into the infusion bag or bottle, mixing well the first liquid composition with second aqueous composition (e.g., gently inverting the infusion bag or bottle containing the composition by hand) to obtain a clear infusion solution free of precipitates

The first step and the second step of the preparation could be done in parallel or any different orders. Thereby the infusion formulation containing the desired dosage of docetaxel obtained in step 4 can be administered to subjects in need thereof. For example, at a dosage from about 50 mg/m² to about 150 mg/m² of docetaxel based on the individual patient' s body surface area ( "BSA" ). In some embodiments, the formulation may be administered at about 50 mg/m², about 60 mg/m², about 75 mg/m², about 90 mg/m², about 100 mg/m², or about 120 mg/m² of docetaxel.In some embodiments, the formulation may be administered at about 75 mg/m² of docetaxel.

In some embodiments, the kit further comprises instructions for use in preparing the infusion formulation (compositions) described herein.

### EXAMPLEs

### Materials and methods

### Winthrop (docetaxel) injection, an authorized generic drug of Taxotere^{®} (reference product):

Winthrop (docetaxel) injection was obtained from Winthrop U.S., a business of Sanofi-Aventis U.S. as 20 mg/mL solution of docetaxel in ethanol and polysorbate 80. One vial of Winthrop (docetaxel) injection contains 20 mg docetaxel (anhydrous), 540 mg of polysorbate 80, and 395 mg of dehydrated ethanol (50% v/v) solution, with citric acid for pH adjustment. In this study, each patient received the final infusion of the reference product at a concentration of 0.3 mg/ml docetaxel and a dose of 75 mg/m². Taking a patient with a BSA of 2 m² as an example, the preparation method of the reference product is as follows:
- For a cancer patient with BSA of about 2 m², the dose of docetaxel in the infusion bag is about 150 mg. To prepare the final infusion solution with a concentration of 0.3mg/ml docetaxel, 7.5ml Winthrop (docetaxel) injection (20mg docetaxel/ml) and 492.5ml 0.9% sodium chloride injection are used in the preparation.
- Using only a 21-gauge needle, aseptically withdraw 7.5ml of Winthrop (docetaxel) injection (20 mg docetaxel/ml) with a calibrated syringe and inject via a single injection (one shot) into the infusion bag or bottle containing 492.5ml of 0.9% Sodium Chloride solution to produce a final concentration of 0.3 mg/ml docetaxel infusion solution. Thoroughly mix the infusion solution by gentle manual rotation.
- The docetaxel infusion solution should be administered intravenously as a 1-hour infusion under ambient room temperature (below 25 °C) and lighting conditions.

### BH009 (test product) :

BH009 was developed as a polysorbate 80-free formulation of docetaxel injection. BH009 is manufactured as an injectable sterile product (20 mg/ml solution of docetaxel in ethanol). In this BH009 drug kit, there is a vial containing 80mg (docetaxel)/4ml of BH009 drug (docetaxel injection), and another vial containing a commercially available 10g infusion of 20% human albumin solution (50ml).Each 4ml of BH009 drug product (Docetaxel Injection) contains 80 mg docetaxel (anhydrous) and 3156 mg dehydrated ethanol, with citric acid for pH adjustment. In this clinical study, each patient received the infusion solution of the test product at a concentration of 0.25 mg/ml docetaxel and at a dose of 75 mg/m². In preparation of the infusion solution of test product, the ratio between BH009 drug product (Docetaxel Injection), 20% Human Albumin Solution, and 0.9% sodium chloride injection used in the preparation is 1:7.5:71.5 (v/v). Taking a patient with a BSA of 2 m² as an example, the preparation method of the test product is as follows:
- For a cancer patient with BSA of about 2 m², the dose of docetaxel in the infusion bag is about 150 mg. To prepare the infusion solution of test product with a concentration of 0.25 mg/ml docetaxel, 7.5ml BH009 (20 mg docetaxel/ml), 56.25 ml diluent (20% Human Albumin Solution), and 536.25 ml 0.9% sodium chloride injection are used in the preparation.
- 56.25 ml diluent (20% Human Albumin Solution) is added into the infusion bag or bottle contained with 536.25 ml of 0.9% Sodium Chloride solution. Thoroughly mix the Albumin - saline solution by gentle manual inversion of the infusion bag or bottle for about 10 times.
- Using only a 21 gauge needle, aseptically withdraw 7.5 ml BH009 drug product (20 mg docetaxel/mL) with a calibrated syringe and inject via a single injection (one quick shot) into the infusion bag or bottle with the prepared Albumin - saline infusion solution to produce a final concentration of 0.25 mg/ml. After injection, immediately thoroughly mix the infusion by gentle manual inversion of the infusion bag or bottle for about 20 times (mixing takes total approximately 1 minute). The infusion solution obtained is a clear aqueous.
- The BH009 infusion solution (test product) should be administered intravenously as a 1-hour infusion under ambient room temperature (below 25 °C) and lighting conditions.

The plasma concentrations of Unbound Docetaxel and Total Docetaxel will be quantified by using validated two separate analytical method respectively, see below.

### The validated method of Determination of Total Docetaxel in K3EDTA Human Plasma by Using LC-ESI-MS/MS:

A liquid chromatographic-tandem mass spectrometric (LC-MS/MS) method for the estimation of Total Docetaxel in human plasma has been developed using Liquid Liquid Extraction technique. This method made use of Turbo Ion Spray (TIS) ionization in positive mode for Docetaxel using triple quadrupole mass spectrometry. Where Docetaxel-d9 was used as an internal standard (IS). Docetaxel was recovered by extraction solvent and subsequently separated on Gemini 5µm C18, 150×4.6 mm using Mobile Phase Buffer (Acetic acid in Water, 0.1 %v/v) : Methanol, 30:70v/v under binary gradient condition, at flow rate of 1.000 mL/minute. Quantification of Docetaxel and Docetaxel-d9 was performed using multiple-reaction monitoring mode (MRM). The method is suitable for the determination of Total Docetaxel in K₃EDTA human plasma over the range from 10.000 ng/mL (LLOQ) to 6000.000 ng/mL (ULOQ) using 0.200 mL of human plasma. The method is validated for analysis of sample diluted by 10 folds (up to 27000.000 ng/mL) of samples.

### The validated method of Determination of Free Docetaxel in K3EDTA Human Plasma by Using LC-ESI-MS/MS:

A liquid chromatographic-tandem mass spectrometric (LC-MS/MS) method for the estimation of Free Docetaxel in human plasma has been developed using Liquid Liquid Extraction technique. This method made use of Turbo Ion Spray (TIS) ionization in positive mode for Docetaxel using triple quadrupole mass spectrometry. Where Docetaxel-d9 was used as an internal standard (IS). The plasma samples were filtered using the Centrifree^{®} Ultrafiltration Device (Centrifugal Filters 1.0 mL, 30KDa) for direct measurement of the unbound concentration of docetaxel. Docetaxel was recovered by extraction solvent and subsequently separated on Gemini 5µm C18, 150x4.6 mm using Mobile Phase Buffer (Acetic acid in Water, 0.1 %v/v) : Methanol, 30:70v/v under isocratic condition, at flow rate of 1.000 mL/minute. Quantification of Docetaxel and Docetaxel-d9 was performed using multiple-reaction monitoring mode (MRM).The method is suitable for the determination of Free Docetaxel in K₃EDTA human plasma over the range from 1.000 ng/mL (LLOQ) to 600.000 ng/mL (ULOQ) using 0.200mL of human plasma. The method is validated for analysis of sample diluted by 10 folds (up to 8000.000 ng/mL) of samples.

### Example 1

A multicenter, open label, randomized, balanced, two treatment, two period, two sequence, crossover, single dose, bioequivalence study was performed wherein patients received either treatment with test product BH009 or the reference product Taxotere^{®} (docetaxel) injection or an approved generic drug of Taxotere^{®}.

### Patient selection criteria

Inclusion criteria: Patients of either gender, ≥18 years of age, were histologically or cytologically confirmed to have solid tumors. The patients are either scheduled to receive treatment with single-agent docetaxel (Taxotere^{®} or an approved generic drug of Taxotere^{®}) at the dose of 75 mg/m², or are already receiving the same single-agent docetaxel at the dose of 75 mg/m². The patients were scheduled to two more cycles of docetaxel treatment, at the same dose, as per the actual treatment plan. The patients had ECOG (Eastern Cooperative Oncology Group) performance stage 0-1, adequate hematopoietic, renal and liver function (patients had to have ANC 1500/mm³, platelet count 100,000/mm³, haemoglobin > 9.0 g/dl, liver function: ALT/AST ≤1.5 × ULN, alkaline phosphatase ≤ 2.5 × ULN, total bilirubin ≤ ULN, renal function: serum creatinine ≤1.5 × ULN), and prothrombin time, international normalized ratio or activated partial thromboplastin time <1.5 × ULN. Use of full dose anticoagulants was permitted. Patients have recovered from adverse events related to prior anticancer therapy (except alopecia and endocrinopathies controlled with hormone replacement therapy) to grade 0-1 (as per CTCAE 5.04 criteria). Exclusion criteria: Patients with a history of hypersensitivity or idiosyncratic reaction to docetaxel, its excipients, and/or related substances including polysorbate 80, paclitaxel, alcohol, dexamethasone and antiemetic (granisetron or ondansetron), or severe cardiovascular disease, an active infection (e.g., tuberculosis, sepsis, or opportunistic infections), severe pleural effusion, and/or peripheral neuropathy ≥grade 2 , hepatitis B surface antigen and hepatitis C (HCV) virus positive, HIV-infected, known brain metastases or neurological symptoms due to brain metastases; Pregnant or lactating women, etc were not included in the study.

### Study design

46 patients were included in the clinical study. Period I (Day 1): Patients will receive 75 mg/m² dose of docetaxel injection for infusion (either test or reference product) on the first day of the chemotherapy cycle. Period II (Day 22): Patients will be crossover to another treatment arm to receive 75 mg/m² dose of docetaxel injection for infusion (either test or reference product depending of crossover sequence) on the first day of the next chemotherapy cycle. All patients should be premedicated with oral corticosteroids such as dexamethasone 16 mg per day (e.g., 8 mg twice daily) for 3 days starting 1 day prior to docetaxel administration in order to reduce the incidence and severity of fluid retention as well as the severity of hypersensitivity reactions. Prophylactic Granisetron (Antiemetic) 2 mg IV or Ondansetron (Antiemetic) 4-8 mg IV (approximately 30 minutes prior) should be given to all the patients before IMP administration in both the periods. Prophylactic antiallergy treatment and G-CSF are permitted at the discretion of the Investigator. The prophylactic treatment and dose should be same prior to dosing in both periods. At 0h before intravenous infusion, 0.500h, 0.667h, 0.833h, 1.000h after infusion (immediately at the end of infusion), After infusion, 6mL of venous blood was collected at 0.083, 0.167, 0.333, 0.500, 1.000, 2.000, 3.000, 6.000, 8.000, 12.000, 24.000 and 48.000h, respectively, for pharmacokinetic analysis. The blood samples of 6 mL each were drawn at 0h before intravenous infusion,0.500h, 0.667h, 0.833h, 1.000h after infusion (immediately at the end of infusion), and at 0.083, 0.167, 0.333, 0.500, 1.000, 2.000, 3.000, 6.000, 8.000, 12.000, 24.000 and 48.000h after the end of the docetaxel infusion, for pharmacokinetic analysis. Plasma concentration of total docetaxel and free docetaxel were quantified using two separate validated analytical methods. Employing the estimated concentration time profiles of free docetaxel and total docetaxel following variables were calculated: Primary variables: Cₘₐₓ, AUC₀₋ₜ and AUC_{0-∞}; Secondary variable: Tmax, t_{1/2}, Kₑₗ, Vd, CL and AUC_{_%Extrap_obs}.

For free docetaxel and total docetaxel, based on the statistical results of 90% confidence intervals for the geometric least square mean ratio (Test product (T)/Reference product (R)) for the pharmacokinetic parameters Cₘₐₓ, AUC₀₋ₜ and AUC_{0-∞}, conclusions were drawn whether test formulation is bioequivalent to reference formulation. Acceptance range for bioequivalence is 80.00% - 125.00% for 90% confidence intervals of the geometric least square means ratio (T/R) for Cₘₐₓ, AUC₀₋ₜ and AUC_{0-∞}.

### Study results

A total of 52 patients were screened during the study. Out of 52 screened patients, 46 patients were randomized during the study, all of them (N=46) were included in the safety population (see **Table 1**). A total of 5 patients dropped out of the clinical study, and 41 patients completed both study periods.

Samples collected from 46 subjects were analyzed for Total Docetaxel and Free Docetaxel Plasma concentrations. According to the requirements of statistical analysis, pharmacokinetic population (N=41) included all the subject who completed the study as per the study protocol. The Pharmacokinetic Parameters of total Docetaxel and Free Docetaxel are shown in **Table 2** and **Table 3.**

Since the pre-dose concentration of one patient in the 41 patients exceeded 5% of Cₘₐₓ, met the elimination criteria specified in the statistical analysis, hence this Patient was excluded from the statistical analysis for bioequivalence evaluation. Therefore, 40 patients were finally included in the BE analysis set. The results of bioequivalence analysis for Total Docetaxel and Free Docetaxel Plasma concentrations are shown in **Table 4** and **Table 5.**

The results of the study found that the test product BH009 and the reference product Winthrop (docetaxel) Injection (an authorized generic drug of Taxotere^{®}) are bioequivalent in patients with advanced solid tumors. The calculated 90% confidence interval for AUC₀₋ₜ, AUC_{0-∞} and Cₘₐₓ of total docetaxel was 91.89% - 107.18%, 91.10% - 106.04%, and 92.18% - 108.00%, respectively, and the calculated 90% confidence interval for AUC₀₋ₜ, AUC_{0-∞} and Cₘₐₓ of free docetaxel was 89.98% - 103.15%, 90.24% - 102.75%, and 85.22% - 101.70%, respectively. AUC₀₋ₜ, AUC_{0-∞} and Cₘₐₓ of both total docetaxel and free docetaxel fell within the bioequivalence (BE) range (80%-125%).

The safety data found that BH009 is well tolerated in the patients, and its hematological toxicity is significantly lower than that of Reference Product Winthrop (docetaxel) Injection (see **Table 6**). The most frequent Grade 3/4 toxicities - hematological toxicities, including neutropenia, anaemia, lymphocytopenia, and leukocytopenia, - were observed with higher frequency in the Reference Product group (Winthrop docetaxel injection) with 15.9% compared to the Test Product group (BH009) with 2.3%, especially leukopenia and neutropenia (6.8% vs 0.0%, 4.6% vs 0.0%). Rates of febrile neutropenia were 0.0% in the study.

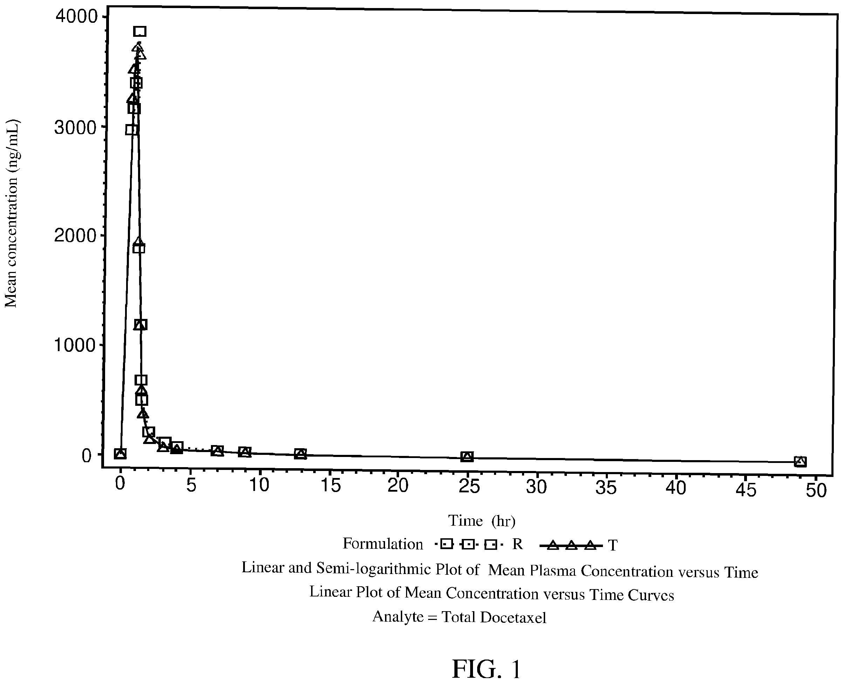

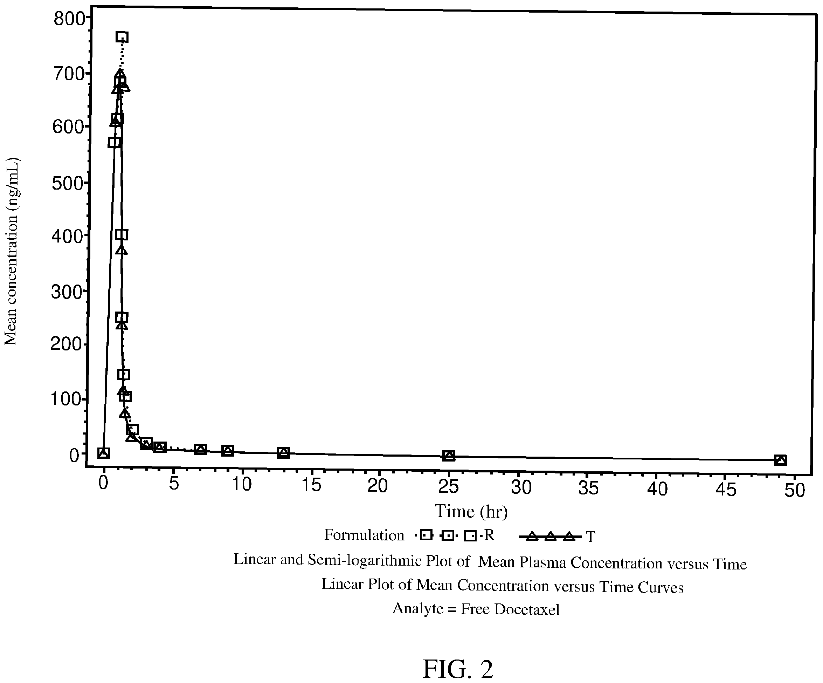

A linear plot of mean concentration of total Docetaxel versus time curves after administration of BH009 and Winthrop (docetaxel) Injection to advanced solid tumor patients is shown in **FIG. 1****.** A linear plot of mean concentration of free Docetaxel versus time curves after administration of BH009 and Winthrop (docetaxel) Injection to advanced solid tumor patients is shown in **FIG. 2****.**

The demographic profile of patients population, measured pharmacokinetic parameters, the BE results for primary pharmacokinetic parameters of Total Docetaxel & Free Docetaxel, and safety statistics are summarized below.

**Table 1: Demographic Profile of safety Population**

| Number of Patients (Safety Population) | Test\Reference | Reference\Test |
|---|---|---|
| | 24 | 22 |
| Gender | | |
| Male | 9(37.5) | 8 (36.4) |
| Female | 15(62.5) | 14(63.6) |

| Age (years) | | |
|---|---|---|
| Mean ± SD | 52.375 ± 9.8193 | 50.909 ± 13.3449 |
| Range (Min / Max) | 33.00 - 73.00 | 32.00 - 83.00 |

| Height (cm) | | |
|---|---|---|
| Mean ± SDs | 159.042 ± 9.9454 | 156.045 ± 7.4417 |
| Range (Min / Max) | 143.00 - 179.00 | 144.00 - 169.00 |

| Weight (kg) | | |
|---|---|---|
| Mean ± SD | 56.571 ± 11.7633 | 54.725 ± 11.3455 |
| Range (Min / Max) | 43.80 - 87.50 | 38.00 - 80.20 |

| BSA (m²) | | |
|---|---|---|
| Mean ± SD | 1.565 ± 0.1405 | 1.525 ± 0.1511 |
| Range (Min / Max) | 1.32- 1.88 | 1.25 - 1.87 |

**Table 2: The Pharmacokinetic Parameters of total Docetaxel for Test product BH009 and Reference Product Winthrop (docetaxel) Injection (N=41 Patients)**

| Parameters (Units) | MEAN ± SD (%CV) | |
|---|---|---|
| | Winthrop (docetaxel) Injection | BH009 |
| Cₘₐₓ (ng/mL) | 3933.7 ± 1377.7 (35.02%) | 3997.0 ± 1530.8 (38.30%) |
| AUCₒ₋ₜ(ng.h/mL) | 3810.7 ± 1501.4 (39.40%) | 3883.8 ± 1839.4 (47.36%) |
| AUC_{0-∞} (ng.h/mL) | 4062.8 ± 1568.6 (38.61%) | 4107.4 ± 1923.4 (46.83%) |

**Table 3: The Pharmacokinetic Parameters of free Docetaxel for Test product BH009 and Reference Product Winthrop (docetaxel) Injection (N=41 Patients)**

| Parameters (Units) | MEAN ± SD(%CV) | |
|---|---|---|
| | Winthrop (docetaxel) Injection | BH009 |
| Cₘₐₓ (ng/mL) | 798.8 ± 263.7 (33.01%) | 756.6 ± 263.8 (34.87%) |
| AUCₒ₋ₜ(ng.h/mL) | 812.4 ± 269.8 (33.21%) | 780.1 ± 317.8 (40.74%) |
| AUC_{0-∞} (ng.h/mL) | 863.7 ± 273.9 (31.71%) | 829.5 ± 324.2 (39.09%) |

**Table 4: The BE results for primary pharmacokinetic parameters of Total Docetaxel for Test product BH009 and Reference Product Winthrop (docetaxel) Injection (N=40 Patients)**

| PK Parameters (Units) | Geometric Least Squares Means and its ratio (N = 40) | | | Intra Patient %CV | 90% Confidence Interval | Power (%) |
|---|---|---|---|---|---|---|
| | Test Product (T) | Reference Product (R) | (T/R) % | | | |
| Cₘₐₓ (ng/mL) | 3758.7 | 3767.1 | 99.78 | 21.14 | 92.18% -108.00% | 99.81 |
| AUC₀₋ₜ (hr*ng/mL) | 3590.7 | 3618.1 | 99.24 | 20.53 | 91.89% -107.18% | 99.87 |
| AUC_{0-∞} (hr*ng/mL) | 3811.5 | 3877.9 | 98.29 | 20.25 | 91.10% -106.04% | 99.89 |

**Table 5: The BE results for primary pharmacokinetic parameters of Free Docetaxel for Test product BH009 and Reference Product Winthrop (docetaxel) Injection (N=40 Patients)**

| PK Parameters (Units) | Geometric Least Squares Means and its ratio (N = 40) | | | Intra Patien t %CV | 90% Confidence Interval | Power (%) |
|---|---|---|---|---|---|---|
| | Test Product (T) | Reference Product (R) | (T/R)% | | | |
| Cₘₐₓ (ng/mL) | 720.6 | 774.1 | 93.10 | 23.67 | 85.22% -101.70% | 99.31 |
| AUC₀₋ₜ (hr*ng/mL) | 750.8 | 779.4 | 96.34 | 17.23 | 89.98% - 103.15% | 99.99 |
| AUC_{0-∞} (hr*ng/mL) | 801.290 | 832.131 | 96.29 | 16.36 | 90.24% - 102.75% | 100.00 |

**Table 6: The safety statistics of test products BH009 and reference products winthrop docetaxel injection**

| **Adverse Reaction** | **Test Product group (n=43)** | | **Reference Product group (n=44)** | |
|---|---|---|---|---|
| | Any | Grage 3/4 | Any | Grage 3/4 |
| **Blood and lymphatic system disorders** | | | | |
| Anaemia | 4 (9.30%) | 1 (2.33%) | 2 (4.55%) | 1(2.27%) |
| Leukopenia | 5 (11.63%) | 0 (0%) | 7 (15.91%) | 3 (6.82%) |
| Neutropenia | 4 (9.30%) | 0 (0%) | 8 (18.18%) | 2 (4.55%) |
| Lymphopenia | 0 (0%) | 0 (0%) | 1 (2.27%) | 1 (2.27%) |

| **Skin and subcutaneous tissue disorders** | | | | |
|---|---|---|---|---|
| Alopecia | 10 (23.26%) | 0 (0%) | 8 (18.18%) | 0 (0%) |
| Rash | 1 (2.33%) | 0 (0%) | 2 (4.55%) | 0 (0%) |

| **Gastrointestinal disorders** | | | | |
|---|---|---|---|---|
| Stomatitis | 1 (2.33%) | 0 (0%) | 1 (2.27%) | 0 (0%) |
| Aphthous ulcer | 0 (0%) | 0 (0%) | 1 (2.27%) | 0 (0%) |
| Abdominal pain | 3 (6.98%) | 0 (0%) | 1 (2.27%) | 0 (0%) |
| Nausea | 8 (18.60%) | 0 (0%) | 9 (20.45%) | 0 (0%) |
| Vomiting | 3 (6.98%) | 0 (0%) | 6 (13.64%) | 0 (0%) |
| Diarrhoea | 11 (25.58) | 0 (0%) | 8 (18.18%) | 0 (0%) |

| **General disorders and administration site conditions** | | | | |
|---|---|---|---|---|
| Pain | 5 (11.63%) | 0 (0%) | 4 (9.09%) | 0 (0%) |
| Asthenia | 10 (23.26%) | 0 (0%) | 15 (34.09%) | 0 (0%) |
| Fatigue | 4 (9.30%) | 0 (0%) | 2 (4.55%) | 0 (0%) |
| Injection site hypoaesthesia | 0 (0%) | 0 (0%) | 1 (2.27%) | 0 (0%) |
| Pyrexia | 6 (13.95%) | 0 (0%) | 8 (18.18%) | 0 (0%) |

| **Nervous system disorders** | | | | |
|---|---|---|---|---|
| Headache | 6 (13.95%) | 0 (0%) | 3 (6.82%) | 0 (0%) |

| **Musculoskeletal and connective tissue disorders** | | | | |
|---|---|---|---|---|
| Arthralgia | 5 (11.63%) | 0 (0%) | 7 (15.91%) | 0 (0%) |
| Neck pain | 1 (2.33%) | 0 (0%) | 1 (2.27%) | 0 (0%) |

| **Metabolism and nutrition disorders** | | | | |
|---|---|---|---|---|
| Decreased appetite | 0 (0%) | 0 (0%) | 3 (6.82%) | 0 (0%) |
| Hyponatraemia | 1 (2.33%) | 0 (0%) | 0 (0%) | 0 (0%) |

| **Investigations** | | | | |
|---|---|---|---|---|
| Haemoglobin decreased | 0 (0%) | 0 (0%) | 1 (2.27%) | 0 (0%) |

### OTHER EMBODIMENTS

It is to be understood that while the present application has been described in conjunction with the detailed description thereof, the foregoing description is intended to illustrate and not limit the scope of the present application, which is defined by the scope of the appended claims. Other aspects, advantages, and modifications are within the scope of the following claims.

## Claims

1. A pharmaceutical composition for infusion comprising docetaxel and human serum albumin,
wherein the composition is prepared by injecting a first liquid composition comprising docetaxel and ethanol into an infusion bag or bottle containing a second aqueous composition comprising human serum albumin in a parenterally acceptable vehicle,
wherein the composition does not contain polysorbate 80,
wherein the composition is administered by infusion to treat a patient diagnosed with a solid tumor selected from breast cancer, non-small cell lunch cancer, prostate cancer, gastric cancer, and head and neck cancer,
wherein the dose of docetaxel infused to said patient in need thereof is about 75 mg/m², and
wherein the composition is prepared within 24 hours prior to being infused to said patient.

2. The pharmaceutical composition of claim 1, wherein the composition is prepared within 4 hours prior to being infused to said patient.

3. The pharmaceutical composition of claim 1, wherein the concentration of docetaxel in the composition is from about 0.05 mg/ml to about 0.5 mg/ml.

4. The pharmaceutical composition of claim 1, wherein the concentration of human serum albumin in the composition is from about 0.01% to about 20% (w/v).

5. The pharmaceutical composition of claim 1, wherein the composition is a clear aqueous infusion solution.

6. The pharmaceutical composition of claim 1, wherein the composition is administered repeatedly to said patient as a new cycle every 3 weeks.

7. The pharmaceutical composition of claim 6, wherein the composition is administered at a dose of 75 mg/m² of docetaxel.

8. The pharmaceutical composition of claim 1, wherein the composition is administered to treat said patient diagnosed with prostatic cancer.

9. The pharmaceutical composition of claim 1, wherein the composition is administered to treat said patient diagnosed with breast cancer.

10. The pharmaceutical composition of claim 1, wherein the composition is administered to treat said patient diagnosed with non-small cell lung cancer.

11. The pharmaceutical composition of claim 1, wherein the composition is administered to treat said patient diagnosed with gastric cancer.

12. The pharmaceutical composition of claim 1, wherein the composition is administered to treat said patient diagnosed with head and neck cancer.
